(19) Europäisches Patentamt
European Patent Office
Office européen des brevets

(11) Veröffentlichungsnummer: **0 036 895**
**A1**

(12) # EUROPÄISCHE PATENTANMELDUNG

(21) Anmeldenummer: **80101479.6**

(22) Anmeldetag: **20.03.80**

(51) Int. Cl.$^3$: **C 07 C 125/065**
C 07 C 125/073

(43) Veröffentlichungstag der Anmeldung:
**07.10.81 Patentblatt 81/40**

(84) Benannte Vertragsstaaten:
**AT BE CH DE FR GB IT LU NL SE**

(71) Anmelder: **BASF Aktiengesellschaft**
**Carl-Bosch-Strasse 38**
**D-6700 Ludwigshafen(DE)**

(72) Erfinder: **Merger, Franz, Dr.**
**Max-Slevogt-Strasse 25**
**D-6710 Frankenthal(DE)**

(72) Erfinder: **Platz, Rolf, Dr.**
**Hansastrasse 5**
**D-6800 Mannheim 1(DE)**

(72) Erfinder: **Towae, Friedrich, Dr.**
**Parkstrasse 22**
**D-6700 Ludwigshafen(DE)**

(54) **Verfahren zur Herstellung von aromatischen Urethanen.**

(57) Herstellung von aromatischen Urethanen durch Umsetzung von aromatischen Aminen mit Hydroxylverbindungen, Kohlenmonoxid und Sauerstoff bzw. Nitroverbindungen bei erhöhter Temperatur und erhöhtem Druck mit speziellen Edelmetallkatalysatoren oder Selen- bzw. Schwefelkatalysatoren.

Die nach dem Verfahren der Erfindung herstellbaren aromatischen Urethane sind wertvolle Ausgangsstoffe für die Herstellung von Farbstoffen, Pflanzenschutzmitteln, Pharmazeutika und Polyurethanen.

EP 0 036 895 A1

Croydon Printing Company Ltd

# Verfahren zur Herstellung von aromatischen Urethanen

Die Erfindung betrifft ein neues Verfahren zur Herstellung von aromatischen Urethanen durch Umsetzung von aromatischen Aminen mit Hydroxylverbindungen, Kohlenmonoxid und Sauerstoff bzw. Nitroverbindungen bei erhöhter Temperatur und erhöhtem Druck mit speziellen Edelmetallkatalysatoren oder Selen- bzw. Schwefelkatalysatoren.

Urethane werden technisch üblicherweise durch Umsetzung von Hydroxylverbindungen mit Isocyanaten (Houben-Weyl, Methoden der Organischen Chemie, Band 8, Seiten 141 und 142) oder von Chlorkohlensäureestern mit Aminen (Houben-Weyl, Methoden der Organischen Chemie, Band 8, Seite 138) hergestellt, wobei sowohl die Isocyanate wie auch die Chlorkohlensäureester im allgemeinen durch Phosgenierung der entsprechenden Amine oder Alkohole gewonnen werden. Der Einsatz des hochtoxischen Phosgen bedingt aufwendige Umweltschutzmaßnahmen.

Es ist aus Chem. Letters, Band 1972, Seiten 373 bis 374, bekannt, daß man Anilin mit äquimolaren Mengen an Selen und mit zehnfach molaren Mengen an Triäthylamin und Methylalkohol unter Einleiten von Kohlenmonoxid zu dem Triäthylaminsalz der N-Phenylselenocarbaminsäure und dieses Salz dann in einer zweiten Stufe unter Einleiten von Sauerstoff zu N-Phenylcarbaminsäuremethylester umsetzt. Die Ausbeute von 30 Prozent und die unwirtschaftliche Verfahrensweise mit hohen Triäthylaminüberschüssen sind nachteilig; außerdem entsteht während der Reaktion in stöchiometrischen Mengen Selenwasserstoff, was besondere Sicherheitsmaßnahmen bei der Reaktionsausführung erfordert.

Als Ergebnis entsprechender Untersuchungen über die optimalen Herstellungsbedingungen lehrt J. Org. Chem., Band 28,

WB/BL

Seiten 585 bis 586 (1963), daß Mengen an Anilin zu Schwefel anstelle von Selen im Molverhältnis 1 : 1,05 bis 1,5 und im Molverhältnis Anilin zum Triäthylaminzusatz wie 1 : 0,1 bis 0,5 sowie mit überschüssigem Methanol eine Ausbeute von 24,3 Prozent an N-Phenylcarbaminsäuremethylester ergeben. Es wird ausdrücklich darauf hingewiesen, daß solche Bedingungen optimal sind und diese in Ausbeute, Einfachheit und Wirtschaftlichkeit unbefriedigende Verfahrensweise keine bessere Ausbeute erzielen läßt; zudem entsteht Schwefelwasserstoff in stöchiometrischen Mengen, was ebenfalls erhebliche Sicherheitsmaßnahmen erfordert.

Es wurde nun gefunden, daß man aromatische Urethane durch katalytische Umsetzung eines aromatischen Amins mit einer Hydroxylverbindung, Kohlenmonoxid und einem Oxidationsmittel vorteilhaft erhält, wenn die Umsetzung mit Sauerstoff oder einer Nitroverbindung als Oxidationsmittel und in Gegenwart von

a) Edelmetallkatalysatoren in Gestalt der Edelmetalle und/oder ihrer Oxide, Sulfate, Nitrate, Halogenide und/oder Acetate

oder

b) katalytischen Mengen von selenhaltigen und/oder schwefelhaltigen Katalysatoren zusammen mit einer zusätzlichen basischen Verbindung als Hilfskatalysator

bei einer Temperatur von mindestens 100°C und einem Druck von mindestens 50 bar durchgeführt wird.

Weiterhin wurde gefunden, daß man das Verfahren vorteilhaft ausführt, wenn die Umsetzung in Gegenwart von Edelmetallkatalysatoren in Gestalt der Edelmetalle und/oder ihrer

Oxide, Sulfate, Nitrate, Halogenide und/oder Acetate und zusätzlich von Zinnverbindungen, Titanverbindungen, Eisenverbindungen, Quecksilberverbindungen, Nickelverbindungen, Vanadiumverbindungen, Antimonverbindungen, Manganverbindungen, Kobaltverbindungen und/oder Kupferverbindungen durchgeführt wird.

Die Umsetzung kann für den Fall der Verwendung von Anilin, Methanol, Sauerstoff oder Nitrobenzol durch die folgenden Formeln wiedergegeben werden:

a) 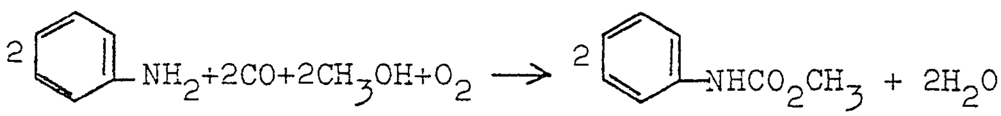

b) 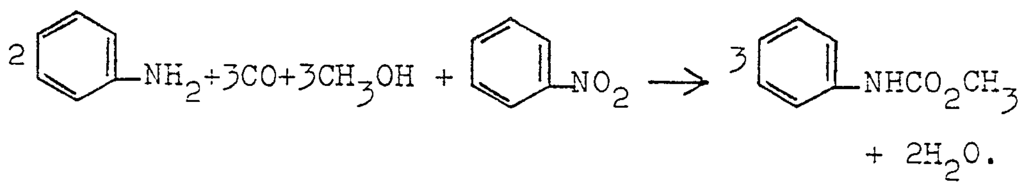

Im Vergleich zu den bekannten Verfahren liefert das Verfahren nach der Erfindung aromatische Urethane in besserer Ausbeute, Raum-Zeit-Ausbeute und Reinheit. Diese vorteilhaften Ergebnisse sind im Hinblick auf den Stand der Technik überraschend. Auch war die erfindungsgemäße Reaktion unter Verwendung von Nitroverbindungen als Oxidationsmittel nicht zu erwarten. Es ist nämlich bekannt, daß organische Nitroverbindungen mit Aminen und Kohlenmonoxid in Gegenwart von tertiären Aminen als Hilfsbase, von Palladiumverbindungen als Katalysatoren und von Xylol als Lösungsmittel bei erhöhter Temperatur und unter Druck in guten Ausbeuten Harnstoffverbindungen liefern (J. Org. Chem., Band 40, Seiten 2 819 bis 2 822 (1975)).

Amin und Hydroxylverbindung können in stöchiometrischer Menge oder im Überschuß des einen, bezogen auf den anderen Ausgangsstoff, vorteilhaft in einer Menge von 1 bis 100,

insbesondere von 5 bis 50 Äquivalenten Hydroxyverbindung je Aminogruppe im Mol Amin, umgesetzt werden. Bevorzugte Urethane sind Monourethane oder Diurethane und solche der Formel

$$R^1\text{-a}\left[\begin{matrix}\text{H}\\ |\\ \text{N-}\underset{\overset{\|}{\text{O}}}{\text{C}}\text{-O-R}^2\end{matrix}\right]_n \qquad \text{I,}$$

bevorzugte Amine sind Monoamine oder Diamine und solche der Formel

$$R^1\text{-a}\left[NH_2\right]_n \qquad \text{II,}$$

bevorzugte Hydroxylverbindungen sind solche der Formel

$$R^2\text{-OH} \qquad \text{III,}$$

worin $R^1$ einen monovalenten oder divalenten aromatischen Rest, vorzugsweise einen unsubstituierten oder durch mehrere, insbesondere 1, 2 oder 3 Alkylgruppen, Alkoxygruppen mit jeweils 1 bis 4 Kohlenstoffatomen, Bromatome, Fluoratome und/oder Chloratome substituierten Phenylrest, Phenylenrest, Diphenylmethanrest, Diphenylen-methanrest, Naphthylrest oder Naphthylenrest bedeutet, $R^2$ einen aliphatischen, araliphatischen, cycloaliphatischen, aromatischen Rest, vorzugsweise einen Alkylrest mit 1 bis 18, insbesondere 1 bis 7 Kohlenstoffatomen, einen Cycloalkylrest mit 5 bis 8 Kohlenstoffatomen, einen Aralkylrest oder Alkylarylrest mit 7 bis 12 Kohlenstoffatomen, einen Phenylrest bezeichnet, n für 1 und a für eine Einfachbindung oder n für 2 und a für 2 Einfachbindungen steht. Die vorgenannten Reste können noch durch unter den Reaktionsbedingungen inerte Gruppen und/oder Atome, z.B. Alkylgruppen oder Alk-

oxygruppen mit jeweils 1 bis 4 Kohlenstoffatomen, substituiert sein.

Folgende Ausgangsstoffe II sind beispielsweise geeignet: Anilin; α- und ß-Naphthylamin; in o-Stellung, m-Stellung oder p-Stellung durch ein Chloratom, Bromatom, die Methyl-, Äthyl-, Propyl-, Isopropyl-, Butyl-, sek.-Butyl-, tert.-Butyl-, Isobutyl-, Methoxy-, Äthoxy-, Propoxy-, Isopropoxy-, Butoxy-, Isobutoxy-, tert.-Butoxy-, sek.-Butoxy-gruppe substituierte Aniline und entsprechend substituierte α- oder ß-Naphthylamine; durch vorgenannte Substituenten in 2,3-, 2,4-, 2,5-, 2,6-, 3,4-, 3,5-Stellung gleich oder unterschiedlich substituierte Aniline und entsprechend substituierte α- oder ß-Naphthylamine; o-, m- oder p-Aminodiphenylmethan, entsprechende Diaminodiphenylmethane und entsprechend durch vorgenannte Substituenten einfach oder zweifach substituierte Monoamino- und Diaminodiphenylmethane; in vorgenannten Stellungen durch eine weitere Aminogruppe einfach oder durch eine Aminogruppe und einen weiteren vorgenannten Substituenten zweifach substituierte Aniline und entsprechend substituierte α- oder ß-Naphthylamine; vorteilhaft sind m- und p-Diaminobenzol, o-, m- und p-Aminotoluol, 2,4- und 2,6-Diaminotoluol, o-, m- und p-Chloranilin, 2,4-, 3,4- und 3,5-Dichloranilin, o-, p-Anisidin, 1,5-Diaminonaphthalin, und alle vorgenannten substituierten oder alle unsubstituierten Diaminodiphenylmethane; besonders bevorzugt sind Anilin, 2,4- und 2,6-Diaminotoluol, 3,5-Dichloranilin, 4,4'- sowie 2,4'-Diaminodiphenylmethan und 1,5-Diaminonaphthalin.

Es kommen z.B. folgende Alkohole als Ausgangsstoffe III in Frage: Methyl-, Äthyl-, Propyl-, Butyl-, Amyl-, Hexyl-, Heptyl-, Octyl-, Nonyl-, Decyl-, Undecyl-, Lauryl-, Tridecyl-, Tetradecyl-, Pentadecyl-, Cetyl-, Margarine-(heptadecyl)-, Stearyl-alkohol, 2-Äthylhexanol, Methylpentanol;

Isobutyl-, sek.-Butyl-, tert.-Butyl-, Isoamyl-, Isohexyl-, Isoheptyl-, Isooctyl-, Isononyl-, Isodecyl-, Isoundecyl-, Isododecyl-, Isotridecyl-, Isotetradecyl-, Isopentadecyl-, Isohexadecyl-, Isoheptadecyl-, Isooctadecyl-alkohol; Äthylenglykolmonoäther und Propylenglykolmonoäther, die durch Umsetzung vorgenannter Fettalkohole mit 10 bis 20 Kohlenstoffatomen mit 2, 3, 4 und 5 Mol Äthylenoxid oder Propylenoxid je Mol Alkohol erhalten werden, sowie entsprechende Glykolmonoäther vorgenannter Fettalkohole nach gleichzeitiger Umsetzung mit Äthylenoxid und Propylenoxid in vorgenannten Molverhältnissen; Methyläthylenglykol, Äthyläthylenglykol, n-Propyläthylenglykol, Isopropyläthylenglykol, n-Butyläthylenglykol, Isobutyläthylenglykol, sek.-Butyläthylenglykol, tert.-Butyläthylenglykol, Methyl-1,2-propylenglykol, Äthyl-1,2-propylenglykol, Cyclohexanol, Benzylalkohol, Cyclopentanol, Cycloheptanol, Phenyläthylalkohol, Phenylpropanol, Phenol, Cyclooctanol, 2-Methylphenol, 3-Methylphenol, 4-Methylphenol, 2-Methoxyphenol, 3-Methoxyphenol, 4-Methoxyphenol, 2,3-Dimethylphenol, 3,4-Dimethylphenol, 2,6-Dimethylphenol, 3,5-Dimethylphenol, 2,3-Dimethoxyphenol, 3,4-Dimethoxyphenol, 3,5-Dimethoxyphenol.

Die Umsetzung wird im allgemeinen bei einer Temperatur von 100 bis 220°C, vorzugsweise von 130 bis 210°C, insbesondere von 140 bis 200°C und bei einem Druck von 50 bis 1000 bar, vorzugsweise von 100 bis 600 bar, diskontinuierlich oder kontinuierlich durchgeführt. Der Druck wird zweckmäßig durch die verwendeten Gase, vorteilhaft Kohlenmonoxid und/oder Luft bzw. Sauerstoff, und dem sich ergebenden Eigendruck des Reaktionsgemischs bei der gewählten Temperatur eingestellt. Man kann in Abwesenheit oder in Gegenwart von unter den Reaktionsbedingungen inerten Lösungsmitteln umsetzen. Vorteilhaft verwendet man die Reaktionskomponenten, z.B. die Hydroxyverbindung, als Lösungsmittel.

Als weitere Lösungsmittel kommen z.B. in Frage: aromatische Kohlenwasserstoffe, z.B. Toluol, o-, m-, p-Xylol, Mesitylen; aromatische Halogenkohlenwasserstoffe, insbesondere Chlorkohlenwasserstoffe, z.B. Chlornaphthalin, Dichlornaphthalin, Chlorbenzol, Fluorbenzol, o-, p- und m-Dichlorbenzol, o-, m-, p-Chlortoluol, 1,2,4-Trichlorbenzol; bevorzugt sind Toluol, Xylole, und entsprechende Gemische. Zweckmäßig verwendet man das Lösungsmittel in einer Menge von 100 bis 10 000 Gewichtsprozent, vorzugsweise von 200 bis 1 000 Gewichtsprozent, bezogen auf Ausgangsstoff II.

Das Kohlenmonoxid wird in stöchiometrischer Menge oder im Überschuß, vorteilhaft in einer Menge von 10 bis 50, insbesondere von 15 bis 30 Mol je Aminogruppe im Mol Ausgangsstoff II umgesetzt.

Die Verfahrensweise a) unterscheidet sich von der Verfahrensweise b) lediglich durch die unterschiedlichen Katalysatoren. Bei der Verfahrensweise a) können Palladium, Platin, Rhodium, Ruthenium, Osmium und/oder Iridium als Einzelmetalle, Legierungen oder als Metallgemische verwendet werden. Ebenfalls ist es möglich, ihre Oxide, Sulfate, Nitrate, Halogenide oder Acetate als Einzelverbindungen oder in Gestalt von Gemischen dieser Derivate desselben Metalls oder in Gestalt von Gemischen der gleichen Derivate unterschiedlicher Metalle oder unterschiedlicher Derivate unterschiedlicher Metalle zu verwenden. Gegebenenfalls kommen auch Gemische entsprechender Verbindungen und Einzelmetalle in Betracht. Bevorzugt sind die Acetate, II-Chloride, IV-Chloride, Bromide, Nitrate, II-Oxide, IV-Oxide, Sulfate des Palladiums, Platins und Rhodiums. Die Edelmetallverbindungen, insbesondere die Halogenide, kommen auch in Gestalt von Komplexverbindungen, z.B. Kaliumtetrachloroplatinat, Kalium-, Natrium-hexachloroplatinat, Kalium- und Natrium-tetrachloropalladat, Kaliumhexachloropalladat, in Betracht. Die Edelmetallkatalysatoren werden in der Regel in katalytischer Menge, vorteilhaft von 0,001 bis 10, insbesondere 0,01 bis 2 Gewichtsprozent Edelmetall (unabhängig von der tatsächlichen Konstitution oder Zusammensetzung des Katalysators), bezogen auf Ausgangsstoff II, verwendet.

In einer bevorzugten Ausführungsform der Verfahrensweise a) gelangen zusätzlich noch vorgenannte Zinn-, Titan-, Eisen-, Quecksilber-, Nickel-, Vanadium-, Antimon-, Mangan-, Kobalt- und/oder Kupferverbindungen als Hilfskatalysatoren zur Anwendung. Vorteilhafte Verbindungen sind die Acetate, Oxide, Nitrate, Phosphate, Sulfate, Tartrate, Fluoride, Molybdate, Bromide, Chloride, Jodide, Oxalate vorgenannter Hilfsmetalle. Bevorzugt sind das Jodid, Bromid, Chlorid, Fluorid, Acetat, Sulfat oder Phosphat von Zinn, Titan, Eisen, Quecksilber, Nickel, Vanadium, Antimon, Mangan, Kobalt oder Kupfer. Besonders bevorzugte Katalysatorkombinationen sind $PdCl_2/FeCl_3$, $PdCl_2/CuCl_2$, $PdCl_2/CoCl_2$, $Pd/CuCl_2$ und $Pd/CoCl_2$, $PdCl_2/MnCl_2$, $PdCl_2/VCl_5$, $PdCl_2HgCl_2$, $Pd/FeCl_3$, $PdCl_2/NiCl_2$, $PdCl_2/SbCl_5$, $PtCl_2/FeCl_3$, $RhCl_2/FeCl_3$, $PtCl_2/CoCl_2$, $RhCl_2/CoCl_2$; besonders bevorzugt sind: $PdCl_2/FeCl_3$, $PdCl_2/CoCl_2$. Die Hilfskatalysatoren werden in der Regel in katalytischen Mengen, vorteilhaft von 0,5 bis 15, insbesondere von 1 bis 10 Gewichtsprozent Hilfsmetall (unabhängig von der tatsächlichen Konstitution oder Zusammensetzung des Hilfskatalysators), bezogen auf Ausgangsstoff II, verwendet.

Bei der Verfahrensweise b) kommen b 1) selenhaltige Katalysatoren, insbesondere Selen als Element oder in Form von Selenverbindungen, in Betracht. Es kommen als Katalysatoren in Frage: Metallselenide, $SeOCl_2$, $TiSe_2$; bevorzugt sind Se, $SeS_2$, COSe. Die selenhaltigen Katalysatoren werden in katalytischen Mengen, vorteilhaft von 0,1 bis 10, insbesondere von 0,5 bis 5 Gewichtsprozent Selen (unabhängig von der tatsächlichen Konstitution oder Zusammensetzung des selenhaltigen Katalysators), bezogen auf Ausgangsstoff II, verwendet.

Bei der Verfahrensweise b) kommen auch b 2) schwefelhaltige Katalysatoren, in der Regel Schwefel als Element oder in Form von Verbindungen des zweiwertigen Schwefels, in Betracht. Als Katalysatoren kommen beispielsweise in Frage: Ammonium-, Natrium- und Kalium-sulfide und -polysulfide; bevorzugt sind elementarer Schwefel, $H_2S$, COS, $Na_2S_x$, $K_2S_x$ (x = 2-6). Die schwefelhaltigen Katalysatoren werden in der Regel in katalytischen Mengen, vorteilhaft von 0,5 bis 20, insbesondere von 3 bis 10 Gewichtsprozent Schwefel (unabhängig von der tatsächlichen Konstitution oder Zusammensetzung des Katalysators), bezogen auf Ausgangsstoff II, verwendet.

Sowohl bei der Verfahrensweise b 1) wie bei b 2) ist eine zusätzliche basische Verbindung als Hilfskatalysator anwesend. Die Umsetzung der Ausführungsform b) wird in der Regel mit einer katalytischen Menge, vorteilhaft mit einer Menge von 1 bis 20, vorzugsweise von 5 bis 15 Gewichtsprozent basischer Verbindung, bezogen auf Ausgangsstoff II, durchgeführt. Bevorzugte basische Verbindungen sind Erdalkali-und Alkalisalze, insbesondere Salze der Phosphorsäure und Alkancarbonsäuren, und insbesondere tertiäre Amine sowie entsprechende Gemische. Es kommen z.B. als basische Verbindungen in Frage: Kaliumphosphat, Natriumphosphat, Lithiumphosphat, Calciumphosphat, Zinkacetat, Natriumformiat, Natriumacetat, Natriumpropionat, Natriumbutyrat, Natriumisobutyrat, Kaliumformiat, Kaliumacetat, Kaliumpropionat, Kaliumbutyrat, Kaliumisobutyrat, Trimethylamin, Triäthylamin, Tripropylamin, Triisopropylamin, Tributylamin, Triisobutylamin, Tri-sek.-butylamin,

Tri-tert.-butylamin, Tribenzylamin, Tricyclohexylamin, Triamylamin, Trihexylamin, N,N-Dimethylanilin, N,N-Diäthylanilin, N,N-Dipropylanilin, N,N-Dimethyltoluidin, N,N-Diäthyltoluidin, N,N-Dipropyltoluidin, N,N-Dimethyl-p-aminopyridin, N,N-Diäthyl-p-aminopyridin, N,N-Diproyl-p-aminopyridin, N-Methylpiperidin, N-Äthylpiperidin, N-Methylpyrrolidin, N-Äthylpyrrolidin, N-Methylimidazol, Pyridin, Isochinolin, Chinolin, α-Picolin, ß-Picolin, γ-Picolin, 2,6-Lutidin, 2,4-Lutidin, bevorzugt sind insbesondere Triäthylamin, Dimethyläthylamin, Methyldiisopropylamin, Pyridin, p-Dimethylaminopyridin, 1,4-Diazabicyclo-[2,2,2]-octan, Diazabicycloundecen, Diazabicyclononen, Lithiumacetat, Natriumacetat, Kaliumacetat.

Die Oxidationsmittel werden vorteilhaft in einem Verhältnis von 0,3 bis 1, vorzugsweise von 0,4 bis 0,6 Mol Nitroverbindung je Aminogruppe im Mol Ausgangsstoff II, im Falle von Sauerstoff, zweckmäßig in Gestalt von Luft als Oxidationsmittel in einem Verhältnis von 0,1 bis 5, vorteilhaft 0,25 bis 2, insbesondere von 0,5 bis 1,5 Mol Sauerstoff je Aminogruppe im Mol Ausgangsstoff II, verwendet. Kommen als Oxidationsmittel organische Nitroverbindungen in Frage, besteht eine vorteilhafte Ausführungsform darin, schon auch zur Vermeidung von Nebenprodukten, die den verwendeten Aminen II entsprechenden Nitroverbindungen zu verwenden. Auf diese Weise wird gegebenenfalls ein Teil oder die Gesamtmenge des Oxidationsmittels zum aromatischen Amin reduziert und dann als Ausgangsstoff II erfindungsgemäß umgesetzt. Geeignete Nitroverbindungen sind daher alle vorgenannten Amine, die jeweils anstelle einer oder mehrerer oder aller Aminogruppen Nitrogruppen tragen. Bevorzugt sind Nitrobenzol, 2,4'- und 4,4'-Dinitrodiphenylmethan, 1,3-Dinitrobenzol, 2,4-Dinitrotoluol, 2,6-Dinitrotoluol, o-Nitrotoluol, p-Nitrotoluol; 3-Nitro-1,2-dimethylbenzol, 4-Nitro-1,2-dimethylbenzol, 2-Nitro-1,3-dimethylbenzol, 4-Nitro-1,3-dimethylbenzol, 5-Nitro-1,3-dimethylbenzol, 1,5-Dinitronaphthalin, o-, m-, p-Nitranilin, p-Chlornitrobenzol; insbesondere Nitrobenzol, 2,6-Dinitrotoluol, 2,4-Dinitrotoluol, 3,5-Dichlornitrobenzol, p-Nitrotoluol.

Die Reaktion kann wie folgt durchgeführt werden: Ein Gemisch von Ausgangsstoff II und III Kohlenmonoxid, Oxidationsmittel, Katalysator a) oder b), gegebenenfalls zusammen mit Hilfskatalysator und/oder Lösungsmittel, wird während 1 bis 3,5 Stunden bei der Reaktionstemperatur und dem Reaktionsdruck gehalten. Aus dem Reaktionsgemisch wird der Endstoff I in üblicher Weise, in der Regel durch Filtration und fraktionierte Destillation oder Kristallisation, abgetrennt.

Die nach dem Verfahren der Erfindung herstellbaren aromatischen Urethane sind wertvolle Ausgangsstoffe für die Herstellung von Farbstoffen, Pflanzenschutzmitteln, Pharmazeutika und Polyurethanen. Bezüglich der Verwendung wird auf die zum Stand der Technik genannten Veröffentlichungen und Ullmanns Encyklopädie der technischen Chemie, Band 5, Seiten 73 bis 76, verwiesen. Man kann aus ihnen durch Abspaltung von Alkoholen entsprechende Isocyanate bzw. durch Umsetzung mit Aminen entsprechende Harnstoffe herstellen (siehe auch deutsche Offenlegungsschrift 26 35 490, US-Patentschrift 3 919 278).

Die in den folgenden Beispielen aufgeführten Teile bedeuten Gewichtsteile. Sie verhalten sich zu den Volumenteilen wie Kilogramm zu Liter.

Beispiel 1

In einem Autoklaven (400 Volumenteile) werden 8,4 Teile Anilin, 100 Teile Äthanol, 0,062 Teile Palladiumchlorid und 0,34 Teile Eisen-(III)-chlorid gegeben. Der Autoklav wird verschlossen und mit 200 bar Kohlenmonoxid und 50 bar Luft beschickt. Dann wird das Gemisch 3 Stunden unter Rühren von 100°C auf 190°C erwärmt, abgekühlt, belüftet und entleert. Nach Abtrennen des Katalysators erhält man (gaschromatographisch bestimmt) 8,6 Teile (82,4% der Theorie) N-Phenyläthylurethan vom Fp 52°C. Der Umsatz beträgt, bezogen auf Ausgangsstoff II, 70 Prozent.

Beispiel 2

Analog Beispiel 1 werden 8,4 Teile Anilin, 100 Teile Äthanol, 0,062 Teile Palladiumchlorid, 0,34 Teile Eisen-(III)-chlorid mit 5,6 Teilen Nitrobenzol zusammengegeben und bei 190°C unter Kohlenmonoxiddruck von 500 bar eine Stunde umgesetzt. Man erhält (gaschromatographisch bestimmt) 8,5 Teile (88,3 % der Theorie) N-Phenyläthylurethan vom Kp 52°C. Der Umsatz beträgt, bezogen auf Ausgangsstoff II, 43 Prozent. 48 Prozent des Nitrobenzols haben sich umgesetzt.

Beispiel 3

Analog Beispiel 2 werden 6,7 Teile 2,4-Diaminotoluol, 100 Teile Äthanol, 0,02 Teile Palladiumchlorid, 0,48 Teile Eisen-(III)-chlorid und 5 Teile 2,4-Dinitrotoluol zusammengegeben und bei 190°C unter einem Kohlenmonoxiddruck von 500 bar 3 Stunden umgesetzt. Man erhält (chromatographisch

bestimmt) 10,8 Teile (49,3 % der Theorie) Toluylen-2,4-diäthylurethan vom Fp 109°C sowie 6,8 Teile eines Gemisches aus Toluylen-2-amino-4-äthylurethan und Toluylen-4-amino-2-äthylurethan. Die Gesamtselektivität auf Urethane beträgt 92 Prozent. Der Umsatz beträgt, bezogen auf Ausgangsstoff II, 100 Prozent. 2,4-Dinitrotoluol hat sich völlig umgesetzt.

Beispiel 4

Analog Beispiel 2 werden 9,3 Teile Anilin mit 13,7 Teilen p-Nitrotoluol, 100 Teilen Äthanol, 0,09 Teilen Palladiumchlorid und 0,5 Teilen Eisen-(III)-chlorid zusammengegeben und bei 190°C unter einem Kohlenmonoxiddruck von 500 bar eine Stunde umgesetzt. Man erhält (gaschromatographisch bestimmt) 9,3 Teile (95,5 % der Theorie) N-Phenyläthylurethan vom Fp 52°C. Der Umsatz beträgt, bezogen auf Ausgangsstoff II, 59 Prozent. 30 Prozent des Nitrotoluols haben sich umgesetzt.

Beispiel 5

9.3 Teile Anilin, 83,7 Teile Äthanol, 0,093 Teile Selen und 0,5 Teile 1,4-Diazabicyclo-[2,2,2]-octan werden in einen Autoklaven gegeben. Der Autoklav wird verschlossen und mit 200 bar Kohlenmonoxid und 50 bar Luft beschickt. Dann wird das Gemisch 2 Stunden unter Rühren von 100°C auf 150°C erwärmt, abgekühlt und entleert. Nach Abtrennen des Katalysators erhält man (gaschromatographisch bestimmt) 11,6 Teile (74 % der Theorie) N-Phenyläthylurethan vom Fp 52°C. Der Umsatz beträgt, bezogen auf Ausgangsstoff II, 95 Prozent.

## Beispiel 6

Analog Beispiel 5 werden 9,3 Teile Anilin, 83,7 Teile Äthanol, 0,1 Teile Schwefel und 0,5 Teile 1,4-Diazabicyclo-[2,2,2]-octan zusammengegeben und 2,5 Stunden unter einem Druck von 200 bar Kohlenmonoxid und 50 bar Luft bei 190°C gehalten. Man erhält (gaschromatographisch bestimmt) 4,6 Teile (71,5 % der Theorie) N-Phenyläthylurethan vom Kp 52°C. Der Umsatz beträgt, bezogen auf Ausgangsstoff II, 39 Prozent.

## Beispiel 7

Analog Beispiel 5 werden 9,3 Teile Anilin, 100 Teile Äthanol, 0,06 Teile Selen und 0,6 Teile 1,4-Diazabicyclo-[2,2,2]-octan zusammengegeben. Anstelle von Luft werden 6,2 Teile Nitrobenzol zugefügt und das Gemisch wird bei 150°C unter einem Kohlenmonoxiddruck von 500 bar 2 Stunden umgesetzt. Man erhält (gaschromatographisch bestimmt) 2,3 Teile (84,5 % der Theorie) N-Phenyläthylurethan vom Kp 52°C. Der Umsatz beträgt, bezogen auf Ausgangsstoff II, 11 Prozent. 24 Prozent des Nitrobenzols haben sich umgesetzt.

## Beispiel 8

Analog Beispiel 7 werden 4 Teile 2,4-Diaminotoluol, 100 Teile Äthanol, 0,15 Teile Selen, 0,3 Teile 1,4-Diazabicyclo-[2,2,2]-octan und 3 Teile 2,4-Dinitrotoluol 2 Stunden lang unter einem Kohlenmonoxiddruck von 300 bar bei 80°C gehalten. Nun werden zusätzlich 50 bar Luft aufgepreßt. Das Gemisch wird für 3 Stunden auf 190°C erhitzt. Man erhält 3,7 Teile (29,9 % der Theorie) Toluylen-2,4-diäthylurethan vom Fp 109°C und 5,6 Teile eines Gemischs aus Toluylen-2-amino-4-äthylurethan und Toluylen-4-amino-

2-äthylurethan. Die Gesamtselektivität auf Urethane beträgt 87 Prozent. Der Umsatz beträgt, bezogen auf Ausgangsstoff II, 96 Prozent. Dinitrotoluol hat sich völlig umgesetzt.

Beispiel 9

Analog Beispiel 7 werden 9,3 Teile Anilin, 100 Teile Äthanol, 0,3 Teile Selen und 0,6 Teile 1,4-Diazabicyclo-[2,2,2]-octan zusammen mit 6,8 Teilen p-Nitrotoluol 2 Stunden lang unter einem Kohlenmonoxiddruck von 500 bar von 100°C auf 150°C erwärmt. Man erhält (chromatographisch bestimmt) 7,1 Teile (71,7 % der Theorie) N-Phenyläthylurethan vom Kp 52°C. Der Umsatz beträgt, bezogen auf Ausgangsstoff II, 60 Prozent.

Beispiel 10

Analog Beispiel 5 werden 9,9 Teile 4,4'-Diamino-diphenylmethan, 100 Teile Methanol, 1 Teil Selen und 2 Teile 1,4-Diazabicyclo-[2,2,2]-octan in eine Autoklaven gegeben. Der Autoklav wird verschlossen und mit 200 bar Kohlenmonoxid und 50 bar Luft beschickt. Dann wird das Gemisch 3 Stunden unter Rühren von 100°C auf 180°C erwärmt, abgekühlt und entleert. Nach Abtrennen des Katalysators erhält man 9,5 Teile (69.3 % der Theorie) 4,4'-Bis-(carbmethoxyamino)-diphenylmethan vom Fp 189°C und 3,3 Teile 4-Amino-4'-carbmethoxyaminodiphenylmethan; die Gesamtselektivität auf Urethane beträgt 98 Prozent. Der Umsatz beträgt, bezogen auf Ausgangsstoff II, 87 Prozent.

Patentansprüche

1. Verfahren zur Herstellung von aromatischen Urethanen durch katalytische Umsetzung eines aromatischen Amins mit einer Hydroxylverbindung, Kohlenmonoxid und einem Oxidationsmittel, dadurch gekennzeichnet, daß die Umsetzung mit Sauerstoff oder einer Nitroverbindung als Oxidationsmittel und in Gegenwart von

a) Edelmetallkatalysatoren in Gestalt der Edelmetalle und/oder ihrer Oxide, Sulfate, Nitrate, Halogenide und/oder Acetate

oder

b) katalytischen Mengen von selen- und/oder schwefelhaltigen Katalysatoren zusammen mit einer zusätzlichen basischen Verbindung als Hilfskatalysator

bei einer Temperatur von mindestens 100°C und einem Druck von mindestens 50 bar durchgeführt wird.

2. Verfahren nach Anspruch 1, dadurch gekennzeichnet, daß die Umsetzung in Gegenwart von Edelmetallkatalysatoren in Gestalt der Edelmetalle und/oder ihrer Oxide, Sulfate, Nitrate, Halogenide und/oder Acetate und zusätzlich von Zinnverbindungen, Titanverbindungen, Eisenverbindungen, Quecksilberverbindungen, Nickelverbindungen, Vanadiumverbindungen, Antimonverbindungen, Manganverbindungen, Kobaltverbindungen und/oder Kupferverbindungen durchgeführt wird.

0036895

Europäisches Patentamt

# EUROPÄISCHER RECHERCHENBERICHT

Nummer der Anmeldung

EP 80 10 1479.6

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| D | CHEMISTRY LETTERS, 1972, Tokyo<br>K. KONDO et al. "A new synthesis of carbamates. The reaction of carbon monoxide with amine and alcohol in the Co-Presence of Selenium and Triethylamine"<br>Seiten 373 bis 374<br>* gesamter Aufsatz *<br>-- | 1 | C 07 C 125/065<br>C 07 C 125/073 |
| | EP - A1 - 0 000 815 (MITSUI TOUTSU CHEMICALS)<br>* Anspruch 1; Seite 3, Absatz 2; Seite 12, Absatz *<br>-- | 1 | RECHERCHIERTE SACHGEBIETE (Int. Cl.³)<br>C 07 C 125/00 |
| | GB - A - 1 472 243 (MITSUI TOATSU CHEMICALS)<br>* Anspruch 1 *<br>-- | 1 | |
| P | EP - A1 - 0 003 989 (BAYER)<br>* Anspruch 1 *<br>---- | 1 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 12-06-1980 | STOOS |